# EUROPEAN PATENT SPECIFICATION

(11) **EP 3 527 201 B1**
(45) Date of publication and mention of the grant of the patent: **18.08.2021**
(21) Application number: 17863062.0
(22) Date of filing: 13.10.2017
(51) Int. Cl.: A61K 9/51, A61K 47/28, A61K 47/26, A61K 47/24, A61K 47/18, A61K 47/34, A61K 31/7088, A61P 35/00

(54) **LIPID NANOPARTICLE FILM MATERIAL COMPOSITION**
LIPIDNANOPARTIKELFILMMATERIALZUSAMMENSETZUNG
COMPOSITION DE MATÉRIAU DE FILM DE NANOPARTICULES LIPIDIQUES

(30) Priority: 17.10.2016 CN 201610902492
(43) Date of publication of application: 21.08.2019
(73) Proprietor: Nanjing Luye Pharmaceutical Co., Ltd., Jiangsu 210061 (CN); Nanjing Asec Nano Biomedicine Co., Ltd., Jiangsu 210061 (CN)
(72) Inventor: CHENG, Guang, Jiangsu 210061 (CN); CHEN, Wenzhong, Jiangsu 210061 (CN); QIN, Lili, Jiangsu 210061 (CN)
(74) Representative: Gong, Jinping
(86) International application number: PCT/CN2017/106032
(87) International publication number: WO 2018/072644

(56) References cited:
- WO-A1-2014/160392
- WO-A1-2015/031536
- WO-A1-2015/090572
- CN-A- 101 732 349
- US-A1- 2013 209 367

## Description

### Technical Field

The invention relates to the technical field of biology, in particular to a lipid nanoparticle membrane composition.

### Background Art

There is no case where Tween is combined with a polyethylene glycol derivative such as TPGS in a lipid nanoparticle membrane of the prior art, for example, use of Tween: short-chain PEG alone, such that the short-chain PEG can increase the repellency between the nanoparticles to prevent the stability from being reduced due to their aggregation. On the other hand, due to the lack of long-chain PEG embedded on the surface of nanoparticles, this nano-preparation is easily phagocytosed by phagocytes and loses its function because Tween is easily lost in the systemic circulation.

Another example is the use of the polyethylene glycol derivative TPGS alone: although it has a long PEG chain, can prevent the recognition of the phagocyte system to a certain extent, does not lose its function for being phagocytosed by phagocytes, and increases the long circulation time. If there are a lot of TPGS, nanoparticles may be difficult to be effectively uptaken by target tumor cells, because of the steric-hindrance effect.

WO 2014/160392 A1 describes a modified docetaxel liposome formulation.

### Summary of the Invention

### 1. Technical problems to be solved by the present invention

A purpose of the present invention is to overcome the above-mentioned technical deficiencies and provide a lipid nanoparticle membrane composition as specified in any of claims 1-15 that is capable of increasing the stability of the nanoparticle itself, thereby promoting the release of a medicament in tumor tissue and reducing the probability of being degraded.

### 2. Embodiments

In order to achieve the above purpose, the embodiment provided by the present invention is as follows:
a lipid nanoparticle membrane composition, the membrane composition comprises a cationic lipid, a neutral phospholipid, cholesterol, Tween, and a polyethylene glycol derivative, with a molar ratio of (25-35):(40-50):(15-25):(1-5):(1-5) in the membrane composition.

In a further embodiment, the cationic lipid comprises: DOTAP, DOTMA, DDAB, or DODMA;
the neutral phospholipid comprises: Egg PC, DOPC, DSPC, DPPC, or DMPC;
the Tween comprises: Tween 20, Tween 40, Tween 60, or Tween 80;
and the polyethylene glycol derivative consists: mPEG-DPPE, mPEG-DMPE, mPEG-DSPE, mPEG-DMG, TPGS, or mPEG-cholesterol.

In a further embodiment, the PEG in the polyethylene glycol derivative comprises monomethyl polyethylene glycol having a molecular weight of 550-5,000.

In a further embodiment, the PEG in the polyethylene glycol derivative comprises monomethyl polyethylene glycol having a molecular weight of 550, 750, 1,000, 2,000, 3,000, and 5,000.

In a further embodiment, the polyethylene glycol derivative is a substance formed by 3,000, and 5,000.

In a further embodiment, the polyethylene glycol derivative is a substance formed by linking a hydrophilic polyethylene glycol chain or a methylated polyethylene glycol chain to a hydrophobic structure that can be embedded into a lipid bilayer; and the hydrophobic structure is cholesterol, vitamin E, dipalmitoylphosphatidylethanolamine, dimyristoylphosphatidylethanolamine, dimyristoylglycerol or a structural analogue.

In a further embodiment, the Tween is Tween 80.

A method for preparing a lipid nanoparticle from a lipid nanoparticle membrane composition, comprises the following steps:
(1) dissolving a cationic lipid, a neutral phospholipid, cholesterol, Tween, and a polyethylene glycol derivative in 80% ethanol in a certain molar ratio to obtain a mixed ethanol solution; dissolving a content in a PBS buffer to obtain a content mixed solution;
(2) mixing the resulting mixed ethanol solution and the content solution in equal volumes to obtain a 40% final ethanol concentration mixed solution;
(3) further diluting the 40% final ethanol concentration mixed solution obtained in step (2) with a PBS solution in an equal volume; repeatedly diluting the final ethanol concentration mixed solution with the PBS solution in an equal volume until a preparation mixed solution with a final ethanol concentration of less than 5% is obtained;
(4) adding a high-salinity solution to the preparation mixed solution obtained in step (3) to obtain a mixed solution containing high concentration salt;
(5) removing ethanol and free uncoated content in the mixed solution obtained in step (4) with an ultrafiltration or dialysis device; and
(6) filtering and sterilizing the product obtained in step (5) through a filter membrane or a filter element with a pore size of less than or equal to 0.22 µm to obtain the lipid nanoparticle.

In a further embodiment, the PBS buffer does not contain DNase and RNase, the specification of the PBS buffer in the step (1) is IX pH=7, and the specification of the PBS buffer in the step (3) is IX pH=7.4.

In a further embodiment, the high-salinity solution is a NaCl solution, and the concentration of NaCl in the mixed solution in step (4) is 0.1-1 M.

In a further embodiment, the concentration of NaCl in the mixed solution is 0.3-0.4 M, preferably 0.3 M.

In a further embodiment, the content is a nucleic acid.

In a further embodiment, the nucleic acid is an oligonucleotide.

In a further embodiment, the ultrafiltration or dialysis device has a molecular weight cutoff of 10,000 to 100,000 daltons.

In a further embodiment, the membrane composition is DOTAP, Egg PC, cholesterol, Tween 80, and TPGS in a molar ratio of 25:45:20:5:5.

In a further embodiment, the membrane composition is DOTMA, Egg PC, cholesterol, Tween 80, and TPGS in a molar ratio of 30:45:20:5:5.

In a further embodiment, the membrane composition is DOTAP, DSPC, cholesterol, Tween 80, and TPGS in a molar ratio of 30:50:20:5:5.

In a further embodiment, the membrane composition is DOTAP, Egg PC, cholesterol,
Tween 80, and mPEG2000-DPPE in a molar ratio of 30:45:20:5:5.
DOTAP: 1,2-dioleoyl-3-trimethylaminopropane chloride salt
DOTMA: 1,2-dioleyloxy-3-trimethylaminopropane chloride salt
DDAB: dioctadecyldimethylammonium bromide
DODMA: 1,2-dioleyloxy-3-dimethylaminopropane
Egg PC: Egg Yolk Phosphatidylcholine
DOPC: Dioleoyl Phosphatidylcholine
DSPC: Distearoyl Phosphatidylcholine
DPPC: Dipalmitoyl Phosphatidylcholine
DMPC: Dimyristoyl Phosphatidylcholine
DPPE: Dipalmitoyl Phosphatidylethanolamine
DMPE: Dimyristoyl Phosphatidylethanolamine
TPGS: D -α-Tocopherol polyethylene glycol succinate

### 3. Beneficial effects

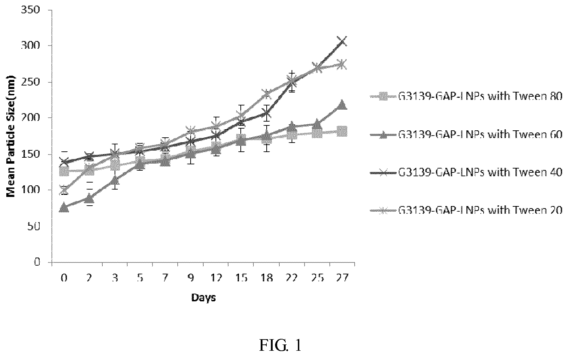

Compared with the prior art, the present invention has the following significant advantages:
1. The nanoparticle membrane of the present invention uses a double PEGylation reagent (Tween and polyethylene glycol derivative) for the first time, which can increase the stability of the nanoparticle itself to a certain extent, promote the release of a medicament in tumor tissue and reduce the possibility of being degraded.
   Tween is often used as a pegylation reagent in the preparation of a nano-preparation. The Tween series has short-chain PEG. The Tween series such as Tween 20/40/60/80 can increase the repellency between nanoparticles in the nano-preparation, make them not easy to aggregate, can improve the stability of the nano-preparation to a certain extent, and has a small increase in particle size for long-term storage.
   The polyethylene glycol derivative reagent comprises mPEG2000-DSPE and TPGS, which can be used as a component of another commonly used nano-preparation, has a long PEG chain which can promote the circulation of nanoparticles in the blood, preventing being uptake by reticuloendothelial cells or phagocytes and lose their function, can prevent the recognition of the immune system to a certain extent, and prolong the circulation time, and a longer PEG chain may influence the uptake of cells to a certain extent.
   The present invention combines the two, the longer PEG chain and the shorter Tween, it was found that the composition of these two pegylation reagents can effectively improve the stability, the long circulation time, the release in target cells, and the ability to degrade genes in target cells of the nanoparticles coated with an antisense oligonucleotide in plasma.
2. The stability test of a composition of Tween series and TPGS at 4 degrees for 27 days according to the present invention, as shown in FIG. 1, shows that in the one-month cycle, the change of the particle size of the nano-preparation is small, indicating that Tween can maintain the overall stability of the preparation.
   The specific experimental data are as follows: in the stability test of Tween 80 at 4 degrees for 27 days, the nano-preparation had a particle size changed from 126 nm to 181 nm, with the most significant stability;
   in the stability test of Tween 20 at 4 degrees for 27 days, the nano-preparation had a particle size changed from 99.8 nm to 274.2 nm, with relatively good stability;
   in the stability test of Tween 40 at 4 degrees for 27 days, the nano-preparation had a particle size changed from 138.5 nm to 305.9 nm, with relatively good stability;
   and in the stability test of Tween 60 at 4 degrees for 27 days, the nano-preparation had a particle size changed from 76.7 nm to 218.6 nm, with relatively good stability.
3. The nano-liposome membrane composition in the present invention comprises a cationic lipid, a neutral phospholipid, cholesterol, Tween, and a polyethylene glycol derivative combined in a certain ratio, so that the prepared nano-liposome can effectively reduce costs, has good stability and high cell uptake, and is a very good nano-carrier.
4. The high-salinity solution added in the preparation process of the present invention can dissociate the free content adsorbed on the surface of the nanoparticles, such as oligonucleotide, and reduce the relatively large particle size generated due to the adsorption of the contents, and the dissociated contents are removed in the subsequent dialysis.
5. The preparation method of the lipid nanoparticles in the present invention adopts a stepwise dilution method with ethanol, however, the method of non-equal-volume on-line mixing used in the prior art is relatively complicated, and two pumps and different types of liquid storage tanks are required, which are not suitable for industrial production. Compared with the prior art, from the dynamics point of view, the present invention is more conducive to the dynamic balance of the two systems and promotes the stability of the subsequent suspension system.

### Brief Description of the Drawings

FIG. 1 is a graph showing the stability test of the composition of Tween series and TPGS at 4 degrees for 27 days according to the present invention.

### Detailed Description of the Invention

The following describes the present invention and its embodiments schematically. The description is not restrictive. The drawings also show only one of the embodiments of the present invention. The actual structure is not limited thereto. Therefore, if those of ordinary skill in the art are inspired by it, without departing from the inventive concept of the present invention, to design a structure and an embodiment similar to the embodiment without creativity, it should belong to the protection scope of the present invention.

### Example 1

A lipid nanoparticle membrane composition, the membrane composition comprises DOTAP, Egg PC, cholesterol, Tween 80, and TPGS, with a molar ratio of 25:45:20:5:5 in the membrane.

A method for preparing a lipid nanoparticle from the above lipid nanoparticle membrane composition comprises the following steps:
(1) dissolving the above substances in 80% ethanol to obtain a mixed ethanol solution; dissolving an oligonucleotide G3139 in a PBS buffer (IX pH=7) to obtain a PBS solution of the oligonucleotide; G3139 is an antisense oligonucleotide consisting of 18 nucleotides, with a nucleotide sequence of 5'-TCT CCC AGC GTG CGC CAT-3';
(2) mixing the obtained mixed ethanol solution and the PBS solution of the oligonucleotide G3139 in equal volumes to obtain a 40% final ethanol concentration mixed solution;
(3) further diluting the 40% final ethanol concentration mixed solution obtained in step (2) with a PBS solution in an equal volume; repeatedly diluting the final ethanol concentration mixed solution with a PBS solution (1×pH=7.4) in an equal volume until a preparation mixed solution with a final ethanol concentration of less than 5% is obtained;
(4) adding a high-salinity solution to the preparation mixed solution obtained in step (3) to obtain a mixed solution with a final concentration of 0.1 M;
(5) removing the ethanol and free antisense oligonucleotides in the mixed solution obtained in step (4) with an ultrafiltration device with a molecular weight cutoff of 10,000 daltons; and
(6) filtering and sterilizing the product obtained in step (5) through a filter membrane through a filter membrane with a pore size of 0.22 µm to obtain the nanoparticles.

### Example 2

A lipid nanoparticle membrane composition, the membrane composition comprises DOTMA, DOPC, cholesterol, Tween 40, and mPEG550-DPPE, with a molar ratio of 35:40:15:1:1 in the membrane.

A method for preparing a lipid nanoparticle from the above lipid nanoparticle membrane composition comprises the following steps:
(1) dissolving the above substances in 80% ethanol to obtain a mixed ethanol solution; dissolving an antisense oligonucleotide G3139 in a PBS buffer (IX pH=7) to obtain a PBS solution of the antisense oligonucleotide G3139;
(2) mixing the obtained mixed ethanol solution and the PBS solution of the antisense oligonucleotide G3139 in equal volumes to obtain a 40% final ethanol concentration mixed solution;
(3) further diluting the 40% final ethanol concentration mixed solution obtained in step (2) with a PBS solution in an equal volume; repeatedly diluting the final ethanol concentration mixed solution with a PBS solution (IX pH=7.4) in an equal volume until a preparation mixed solution with a final ethanol concentration of less than 5% is obtained;
(4) adding a high-concentration salt NaCl solution to the preparation mixed solution obtained in step (3) to obtain a mixed solution with a final concentration of 0.3 M;
(5) removing the ethanol and free antisense oligonucleotides in the mixed solution obtained in step (4) with an ultrafiltration device with a molecular weight cutoff of 50,000 daltons; and
(6) filtering and sterilizing the product obtained in step (5) through a filter membrane through a filter membrane with a pore size of 0.20 µm to obtain the nanoparticles.

### Example 3

A lipid nanoparticle membrane composition, the membrane composition comprises DDAB, DSPC, cholesterol, Tween 60, and mPEG2000-DMPE, with a molar ratio of 30:50:25:3:3 in the membrane.

The method for preparing a lipid nanoparticle from the above lipid nanoparticle membrane composition comprises the following steps:
(1) dissolving the above substances in 80% ethanol to obtain a mixed ethanol solution; dissolving an antisense oligonucleotide in a PBS buffer (IX pH=7) to obtain a PBS solution of the antisense oligonucleotide G3139;
(2) mixing the obtained mixed ethanol solution and the PBS solution of the antisense oligonucleotide G3139 in equal volumes to obtain a 40% final ethanol concentration mixed solution;
(3) further diluting the 40% final ethanol concentration mixed solution obtained in step (2) with a PBS solution in an equal volume; repeatedly diluting the final ethanol concentration mixed solution with a PBS solution (1×pH=7.4) in an equal volume until a preparation mixed solution with a final ethanol concentration of less than 5% is obtained;
(4) adding a high-salinity solution to the preparation mixed solution obtained in step (3) to obtain a mixed solution with a final concentration of 1 M;
(5) removing the ethanol and free antisense oligonucleotide G3139 in the mixed solution obtained in step (4) with an ultrafiltration device with a molecular weight cutoff of 100,000 daltons; and
(6) filtering and sterilizing the product obtained in step (5) through a filter membrane through a filter membrane with a pore size of 0.22 µm to obtain the nanoparticles.

### Example 4

(1) DOTAP/Egg PC/Cholesterol/Tween 80/TPGS=25:50:20:5:5
(2) DOTAP/Egg PC/Cholesterol/Tween 80/TPGS=30:50:20:5:5
(3) DOTAP/Egg PC/Cholesterol/Tween 80/TPGS=35:50:20:5:5

| Preparation | Particle size (nm) | Zeta potential (mV) | G3139 encapsulation rate (%) |
|---|---|---|---|
| 1 | 123.4 | 7.45 | 89.2% |
| 2 | 132.5 | 7.96 | 83.8% |
| 3 | 128.1 | 8.55 | 84.9% |

By adjusting the molar ratio of DOTAP-positive phospholipid and conducting single-factor experiments, it was found that the particle size and encapsulation rate of the first group were significantly better than those of the other two groups under the same conditions of other components, and the zeta potentials remained basically the same, and then the first group was selected as the optimal formula for the following screening.
(1) DOTAP/Egg PC/Cholesterol/Tween 80/TPGS=25:40:20:5:5
(2) DOTAP/Egg PC/Cholesterol/Tween 80/TPGS=25:45:20:5:5
(3) DOTAP/Egg PC/Cholesterol/Tween 80/TPGS=25:50:20:5:5

| Preparation | Particle size (nm) | Zeta potential (mV) | G3139 encapsulation rate (%) |
|---|---|---|---|
| 1 | 138.6 | 7.53 | 83.6% |
| 2 | 123.4 | 7.45 | 89.2% |
| 3 | 136.0 | 7.21 | 84.2% |

By adjusting the molar ratio of Egg PC-phospholipid and conducting single-factor experiments, it was found that the particle size and encapsulation rate of the second group were significantly better than those of the other two groups under the same conditions of other components, and the zeta potential remained basically the same, and then the second group was selected as the optimal formula for the following screening.
(1) DOTAP/Egg PC/Cholesterol/Tween 80/TPGS=25:45:15:5:5
(2) DOTAP/Egg PC/Cholesterol/Tween 80/TPGS=25:45:20:5:5
(3) DOTAP/Egg PC/Cholesterol/Tween 80/TPGS=25:45:25:5:5

| Preparation | Particle size (nm) | Zeta potential (mV) | G3139 encapsulation rate (%) |
|---|---|---|---|
| 1 | 120.4 | 7.78 | 80.4% |
| 2 | 123.4 | 7.45 | 89.2% |
| 3 | 143.4 | 7.03 | 86.2% |

By adjusting the molar ratio of cholesterol and conducting single-factor experiments, it was found that the particle size and encapsulation rate of the second group were significantly better than those of the other two groups under the same conditions of other components, and the zeta potential remained basically the same, and then the second group was selected as the optimal formula for the following screening.
(1) DOTAP/Egg PC/Cholesterol/Tween 80/TPGS=25:45:15:1:5
(2) DOTAP/Egg PC/Cholesterol/Tween 80/TPGS=25:45:20:3:5
(3) DOTAP/Egg PC/Cholesterol/Tween 80/TPGS=25:45:25:5:5

| Preparation | Particle size (nm) | Zeta potential (mV) | G3139 encapsulation rate (%) |
|---|---|---|---|
| 1 | 135.4 | 9.23 | 78.4% |
| 2 | 128.0 | 8.53 | 84.7% |
| 3 | 123.4 | 7.45 | 89.2% |

By adjusting the molar ratio of Tween 80 and conducting single-factor experiments, it was found that the particle size and encapsulation rate of the third group were significantly better than those of the other two groups under the same conditions of other components, and the zeta potential remained basically the same, and then the third group was selected as the optimal formula for the following screening.
(1) DOTAP/Egg PC/Cholesterol/Tween 80/TPGS=25:45:15:5:1
(2) DOTAP/Egg PC/Cholesterol/Tween 80/TPGS=25:45:20:5:3
(3) DOTAP/Egg PC/Cholesterol/Tween 80/TPGS=25:45:25:5:5

| Preparation | Particle size (nm) | Zeta potential (mV) | G3139 encapsulation rate (%) |
|---|---|---|---|
| 1 | 129.2 | 8.53 | 82.6% |
| 2 | 124.6 | 7.91 | 79.3% |
| 3 | 123.4 | 7.45 | 89.2% |

By adjusting the molar ratio of TPGS and conducting single-factor experiments, it was found that the particle size and encapsulation rate of the third group were significantly better than those of the other two groups under the same conditions of other components, and the zeta potential remained basically the same, and then the third group was selected as the optimal formula for the following screening.
(1) DOTMA/Egg PC/Cholesterol/Tween 80/TPGS=25:45:20:5:5
(2) DOTMA/Egg PC/Cholesterol/Tween 80/TPGS=30:45:20:5:5
(3) DOTMA/Egg PC/Cholesterol/Tween 80/TPGS=35:45:20:5:5

| Preparation | Particle size (nm) | Zeta potential (mV) | G3139 encapsulation rate (%) |
|---|---|---|---|
| 1 | 131.4 | 5.76 | 88.4% |
| 2 | 127.2 | 6.66 | 91.9% |
| 3 | 126.4 | 6.75 | 84.6% |

In addition, other phospholipids were also screened. By adjusting the molar ratio of DOTMA-positive phospholipid and conducting single-factor experiments, it was found that the particle size and encapsulation rate of the second group were significantly better than those of the other two groups under the same conditions of other components, and the zeta potential remained basically the same, and then the second group was selected as the optimal formula for the following screening.
(1) DOTAP/DSPC/Cholesterol/Tween 80/TPGS=30:40:20:5:5
(2) DOTAP/DSPC/Cholesterol/Tween 80/TPGS=30:45:20:5:5
(3) DOTAP/DSPC/Cholesterol/Tween 80/TPGS=30:50:20:5:5

| Preparation | Particle size (nm) | Zeta potential (mV) | G3139 encapsulation rate (%) |
|---|---|---|---|
| 1 | 124.4 | 7.12 | 82.8% |
| 2 | 126.0 | 8.04 | 87.1% |
| 3 | 131.8 | 7.71 | 89.9% |

By adjusting the molar ratio of DSPC-phospholipid and conducting single-factor experiments, it was found that the particle size and encapsulation rate of the third group were significantly better than those of the other two groups under the same conditions of other components, and the zeta potential remained basically the same, and then the third group was selected as the optimal formula for the following screening.
(1) DOTAP/Egg PC/Cholesterol/Tween 80/DPPE-mPEG2000=30:45:20:5:1
(2) DOTAP/Egg PC/Cholesterol/Tween 80/DPPE-mPEG2000=30:45:20:5:3
(3) DOTAP/Egg PC/Cholesterol/Tween 80/DPPE-mPEG2000=30:45:20:5:5

| Preparation | Particle size (nm) | Zeta potential (mV) | Encapsulation rate (%) |
|---|---|---|---|
| 1 | 142.6 | 7.68 | 85.2% |
| 2 | 134.5 | 6.63 | 87.3% |
| 3 | 135.8 | 7.90 | 91.4% |

By adjusting the molar ratio of DPPE-mPEG2000 and conducting single-factor experiments, it was found that the particle size and encapsulation rate of the third group were significantly better than those of the other two groups under the same conditions of other components, and the zeta potential remained basically the same.

After the screening of the compositions and ratios of the above formulas, it can be concluded that the formula with the composition and ratio of DOTAP/Egg PC/Cholesterol/Tween 80/TPGS=25:45:20:5:5 has a relatively small and stable particle size and encapsulation rate, as well as a relatively moderate positive zeta potential. In addition, DOTMA/Egg PC/Cholesterol/Tween 80/TPGS=30:45:20:5:5, DOTAP/DSPC/Cholesterol/Tween 80/TPGS=30:50:20:5:5, and DOTAP/Egg PC/ Cholesterol/Tween 80/DPPE-mPEG2000=30:45:20:5:5 are also preferred ratios in the composition of other components in terms of particle size, zeta potential, encapsulation rate, and stability.

The stability test of a composition of Tween series and TPGS at 4 degrees for 27 days according to the present invention, as shown in FIG. 1, shows that in the one-month, the change of the particle size of the nano-preparation is small, indicating that Tween can maintain the overall stability of the preparation.

The specific experimental data are as follows: in the stability test of Tween 80 at 4 degrees for 27 days, the G3139-GAP nano-preparation had a particle size changed from 126 nm to 181 nm, with the most significant stability;
in the stability test of Tween 20 at 4 degrees for 27 days, the G3139-GAP nano-preparation had a particle size changed from 99.8 nm to 274.2 nm, with relatively good stability;
in the stability test of Tween 40 at 4 degrees for 27 days, the G3139-GAP nano-preparation had a particle size changed from 138.5 nm to 305.9 nm, with relatively good stability;
and in the stability test of Tween 60 at 4 degrees for 27 days, the G3139-GAP nano-preparation had a particle size changed from 76.7 nm to 218.6 nm, with relatively good stability.

## Claims

1. A lipid nanoparticle membrane composition, **characterized in that** the membrane composition comprises a cationic lipid, a neutral phospholipid, cholesterol, Tween, and a polyethylene glycol derivative, with a molar ratio of (25-35):(40-50):(15-25):(1-5):(1-5) in the membrane composition; and wherein the cationic lipid comprises: DOTAP, DOTMA, DDAB, or DODMA;
the neutral phospholipid comprises: Egg PC, DOPC, DSPC, DPPC, or DMPC;
the Tween comprises: Tween 20, Tween 40, Tween 60, or Tween 80;
and the polyethylene glycol derivative consist: mPEG-DPPE, mPEG-DMPE, mPEG-DSPE, mPEG-DMG, TPGS, or mPEG-cholesterol.

2. The lipid nanoparticle membrane composition according to claim 1, **characterized in that** the PEG in the polyethylene glycol derivative comprises monomethyl polyethylene glycol having a molecular weight of 550-5,000.

3. The lipid nanoparticle membrane composition according to claim 2, **characterized in that** the polyethylene glycol derivative is a substance formed by linking a hydrophilic polyethylene glycol chain or a methylated polyethylene glycol chain to a hydrophobic structure that can be embedded into a lipid bilayer; and the hydrophobic structure is cholesterol, vitamin E, dipalmitoylphosphatidylethanolamine, dimyristoylphosphatidylethanolamine, dimyristoylglycerol or a structural analogue.

4. The lipid nanoparticle membrane composition according to claim 1, **characterized in that** the Tween is Tween 80.

5. A method for preparing a lipid nanoparticle from a lipid nanoparticle membrane composition, **characterized by** comprising the following steps:
(1) dissolving a cationic lipid, a neutral phospholipid, cholesterol, Tween, and a polyethylene glycol derivative in 80% ethanol in a certain molar ratio to obtain a mixed ethanol solution; dissolving a content in a PBS buffer to obtain a content mixed solution;
(2) mixing the resulting mixed ethanol solution and the content solution in equal volumes to obtain a 40% final ethanol concentration mixed solution;
(3) further diluting the 40% final ethanol concentration mixed solution obtained in step (2) with a PBS solution in an equal volume; repeatedly diluting the final ethanol concentration mixed solution with the PBS solution in an equal volume until a preparation mixed solution with an ethanol final concentration of less than 5% is obtained;
(4) adding a high-salinity solution to the preparation mixed solution obtained in step (3) to obtain a mixed solution containing high concentration salt;
(5) removing ethanol and free uncoated contents in the mixed solution obtained in step (4) with an ultrafiltration or dialysis device; and
(6) filtering and sterilizing a product obtained in step (5) through a filter membrane or a filter element with a pore size of less than or equal to 0.22 µm to obtain the lipid nanoparticle.

6. The method for preparing a lipid nanoparticle from a lipid nanoparticle membrane composition according to claim 5, **characterized in that** the PBS buffer does not contain DNase and RNase, the PBS buffer in the step (1) has a specification of IX pH=7, and the PBS buffer in the step (3) has a specification of IX pH=7.4.

7. The method for preparing a lipid nanoparticle from a lipid nanoparticle membrane composition according to claim 5, **characterized in that** the high-salinity solution is a NaCl solution, and the concentration of NaCl in the mixed solution in step (4) is 0.1-1 M.

8. The method for preparing a lipid nanoparticle from a lipid nanoparticle membrane composition according to claim 7, **characterized in that** the concentration of NaCl in the mixed solution is 0.3-0.4 M.

9. The method for preparing a lipid nanoparticle from a lipid nanoparticle membrane composition according to claim 5, **characterized in that** the content is a nucleic acid.

10. The method for preparing a lipid nanoparticle from a lipid nanoparticle membrane composition according to claim 9, **characterized in that** the nucleic acid is an oligonucleotide.

11. The method for preparing a lipid nanoparticle from a lipid nanoparticle membrane composition according to claim 5, **characterized in that** the ultrafiltration or dialysis device has a molecular weight cutoff of 10,000 to 100,000 daltons.

12. The lipid nanoparticle membrane composition according to claim 1, **characterized in that** the membrane composition comprises DOTAP, Egg PC, cholesterol, Tween 80, and TPGS in a molar ratio of 25:45:20:5:5.

13. The lipid nanoparticle membrane composition according to claim 1, **characterized in that** the membrane composition comprises DOTMA, Egg PC, cholesterol, Tween 80, and TPGS in a molar ratio of 30:45:20:5:5.

14. The lipid nanoparticle membrane composition according to claim 1, **characterized in that** the membrane composition comprises DOTAP, DSPC, cholesterol, Tween 80, and TPGS in a molar ratio of 30:50:20:5:5.

15. The lipid nanoparticle membrane composition according to claim 1, **characterized in that** the membrane composition comprises DOTAP, Egg PC, cholesterol, Tween 80, and mPEG2000-DPPE in a molar ratio of 30:45:20:5:5.

## Patentansprüche

1. Lipidnanopartikel-Membranzusammensetzung, **dadurch gekennzeichnet, dass** die Membranzusammensetzung ein kationisches Lipid, ein neutrales Phospholipid, Cholesterin, Tween und ein Polyethylenglykol-Derivat in einem Molverhältnis von (25-35):(40-50):(15-25):(1-5):(1-5) in der Membranzusammensetzung umfasst;
und wobei das kationische Lipid umfasst: DOTAP, DOTMA, DDAB, oder DODMA;
das neutrale Phospholipid umfasst: Ei-PC, DOPC, DSPC, DPPC oder DMPC; das Tween umfaßt: Tween 20, Tween 40, Tween 60, oder Tween 80;
und das Polyethylenglykol-Derivat bestehen: mPEG-DPPE, mPEG-DMPE, mPEG-DSPE, mPEG-DMG, TPGS oder mPEG-Cholesterin.

2. Lipidnanopartikel-Membranzusammensetzung nach Anspruch 1, **dadurch gekennzeichnet, dass** das PEG im Polyethylenglykol-Derivat einen Monomethyl-Polyethylenglykol mit einem Molekulargewicht von 550-5000 umfasst.

3. Lipidnanopartikel-Membranzusammensetzung nach Anspruch 2, **dadurch gekennzeichnet, dass** das Polyethylenglykol-Derivat eine Substanz ist, die durch Binden einer hydrophilen Polyethylenglykol-Kette oder einer methyliertenPolyethylenglykol-Kette an eine hydrophobe Struktur gebildet wird, die in eine Lipid-Doppelschicht eingebettet werden kann; und die hydrophobe Struktur Cholesterin, Vitamin E, Dipalmitoylphosphatidylethanolamin, Dimyristoylphosphatidylethanolamin, Dimyristoylglycerin oder ein Strukturanalogon ist.

4. Lipidnanopartikel-Membranzusammensetzung nach Anspruch 1, **dadurch gekennzeichnet, dass** das Tween ein Tween 80 ist.

5. Verfahren zur Herstellung eines Lipidnanopartikels aus einer Lipidnanopartikel-Membranzusammensetzung, **dadurch gekennzeichnet, dass** es die folgenden Schritte umfasst:
(1) Lösen eines kationischen Lipids, eines neutralen Phospholipids, Cholesterin, Tween und eines Polyethylenglykol-Derivats in 80%igem Ethanol in einem bestimmten molaren Verhältnis, um eine gemischte Ethanollösung zu erhalten; Lösen eines Inhalts in einem PBS-Puffer, um eine Mischlösung des Inhalts zu erhalten;
(2) Mischen der resultierenden gemischten Ethanollösung und der Inhaltslösung in gleichem Volumen, um eine Mischlösung mit 40%iger Endkonzentration von Ethanolzu erhalten;
(3) weiteres Verdünnen der in Schritt (2) erhaltenen Mischlösung mit 40 %iger Endkonzentration von Ethanol mit einer PBS-Lösung in gleichem Volumen; wiederholtes Verdünnen der Mischlösung mit Endkonzentration von Ethanol mit der PBS-Lösung in gleichem Volumen, bis eine Präparationsmischlösungerhalten wird, bei der dieEndkonzentration von Ethanol weniger als 5 % ist;
(4) Zugeben einer hochsalzhaltigenLösung zu der in Schritt (3) erhaltenen Präparationsmischlösung, um eine Mischlösung mit hoher Salzkonzentration zu erhalten;
(5) Entfernen von Ethanol und freiem, nicht beschichtetem Inhalt in der in Schritt (4) erhaltenen Mischlösung mittels einer Ultrafiltrations- oder Dialysevorrichtung; und
(6) Filtrieren und Sterilisieren eines in Schritt (5) erhaltenen Produkts durch eine Filtermembran oder ein Filterelement mit einer Porengröße von weniger als oder gleich 0,22 µm, um das Lipidnanopartikel zu erhalten.

6. Verfahren zur Herstellung eines Lipidnanopartikels aus einer Lipidnanopartikel-Membranzusammensetzung nach Anspruch 5, **dadurch gekennzeichnet, dass** der PBS-Puffer keine DNase und RNase enthält, der PBS-Puffer in Schritt (1) eine Spezifikation von 1X pH=7 aufweist und der PBS-Puffer in Schritt (3) eine Spezifikation von 1X pH=7,4 aufweist.

7. Verfahren zur Herstellung eines Lipidnanopartikels aus einer Lipidnanopartikel-Membranzusammensetzung nach Anspruch 5, **dadurch gekennzeichnet, dass** die hochsalzhaltige Lösung eine NaCl-Lösung ist und die Konzentration von NaCl in der Mischlösung in Schritt (4) 0,1-1 M beträgt.

8. Verfahren zur Herstellung eines Lipidnanopartikels aus einer Lipidnanopartikel-Membranzusammensetzung nach Anspruch 7, **dadurch gekennzeichnet, dass** die Konzentration von NaCl in der Mischlösung 0,3-0,4 M beträgt.

9. Verfahren zur Herstellung eines Lipidnanopartikels aus einer Lipidnanopartikel-Membranzusammensetzung nach Anspruch 5, **dadurch gekennzeichnet, dass** der Inhalt eine Nukleinsäure ist.

10. Verfahren zur Herstellung eines Lipidnanopartikels aus einer Lipidnanopartikel-Membranzusammensetzung nach Anspruch 9, **dadurch gekennzeichnet, dass** die Nukleinsäure ein Oligonukleotid ist.

11. Verfahren zur Herstellung eines Lipidnanopartikels aus einer Lipidnanopartikel-Membranzusammensetzung nach Anspruch 5, **dadurch gekennzeichnet, dass** die Ultrafiltrations- oder Dialysevorrichtung eine Molekulargewichtsgrenzevon 10.000 bis 100.000 Dalton aufweist.

12. Lipidnanopartikel-Membranzusammensetzung nach Anspruch 1, **dadurch gekennzeichnet, dass** die Membranzusammensetzung DOTAP, Ei-PC, Cholesterin, Tween 80 und TPGS in einem molaren Verhältnis von 25:45:20:5:5 umfasst.

13. Lipidnanopartikel-Membranzusammensetzung nach Anspruch 1, **dadurch gekennzeichnet, dass** die Membranzusammensetzung DOTMA, Ei-PC, Cholesterin, Tween 80 und TPGS in einem Molverhältnis von 30:45:20:5:5 umfasst.

14. Lipidnanopartikel-Membranzusammensetzung nach Anspruch 1, **dadurch gekennzeichnet, dass** dieMembranzusammensetzung DOTAP, DSPC, Cholesterin, Tween 80 und TPGS in einem Molverhältnis von 30:50:20:5:5 umfasst.

15. Lipidnanopartikel-Membranzusammensetzung nach Anspruch 1, **dadurch gekennzeichnet, dass** die Membranzusammensetzung DOTAP, Ei-PC, Cholesterin, Tween 80 und mPEG2000-DPPE in einem Molverhältnis von 30:45:20:5:5 umfasst.

## Revendications

1. Composition membranaire à nanoparticules lipidiques, **caractérisée en ce que** la composition membranaire comprend un lipide cationique, un phospholipide neutre, du cholestérol, du Tween, et un dérivé de polyéthylène glycol, avec un rapport molaire de (25-35):(40-50):(15-25):(1-5):(1-5) dans la composition membranaire; et dans lequel le lipide cationique comprend: DOTAP, DOTMA, DDAB ou DODMA;
le phospholipide neutre comprend: Egg PC, DOPC, DSPC, DPPC ou DMPC;
le Tween comprend: le Tween 20, le Tween 40, le Tween 60 ou le Tween 80;
et le dérivé de polyéthylène glycol consiste en: mPEG-DPPE, mPEG-DMPE, mPEG-DSPE, mPEG-DMG, TPGS ou mPEG-cholestérol.

2. Composition membranaire à nanoparticules lipidiques selon la revendication 1, **caractérisée en ce que** le PEG dans le dérivé de polyéthylène glycol comprend du monométhyl polyéthylène glycol ayant un poids moléculaire de 550 à 5 000.

3. Composition membranaire de nanoparticules lipidiques selon la revendication 2, **caractérisée en ce que** le dérivé de polyéthylène glycol est une substance formée en liant une chaîne polyéthylène glycol hydrophile ou une chaîne polyéthylène glycol méthylée à une structure hydrophobe pouvant être noyée dans une bicouche lipidique ; et la structure hydrophobe est le cholestérol, la vitamine E, la dipalmitoylphosphatidyléthanolamine, la dimyristoylphosphatidyléthanolamine, le dimyristoylglycérol ou un analogue structurel.

4. Composition membranaire à nanoparticules lipidiques selon la revendication 1, **caractérisée en ce que** le Tween est le Tween 80.

5. Procédé de préparation d'une nanoparticule lipidique à partir d'une composition membranaire de nanoparticule lipidique, **caractérisé en ce qu'**il comprend les étapes suivantes:
(1) dissoudre un lipide cationique, un phospholipide neutre, du cholestérol, du Tween et un dérivé de polyéthylène glycol dans 80 % d'éthanol dans un certain rapport molaire pour obtenir une solution d'éthanol mixte; dissoudre un contenu dans un tampon PBS pour obtenir une solution mixte de contenu ;
(2) mélanger la solution mixte d'éthanol résultante et la solution de contenue en volumes égaux pour obtenir une solution mixte à une concentration finale en éthanol de 40%;
(3) diluer davantage la solution mixte à concentration finale en éthanol à 40 % obtenue à l'étape (2) avec une solution de PBS dans un volume égal; diluer à plusieurs reprises la solution mixte à concentration finale en éthanol avec la solution de PBS dans un volume égal jusqu'à ce qu'une solution mixte de préparation avec une concentration finale en éthanol inférieure à 5 % soit obtenue;
(4) ajouter une solution à haute salinité à la solution mixte de préparation obtenue à l'étape (3) pour obtenir une solution mixte contenant une concentration élevée de sel ;
(5) éliminer l'éthanol et le contenu libre non enrobé dans la solution mixte obtenue à l'étape (4) avec un dispositif d'ultrafiltration ou de dialyse ; et
(6) filtrer et stériliser un produit obtenu à l'étape (5) à travers une membrane filtrante ou un élément filtrant de taille de pores inférieure ou égale à 0,22 µm pour obtenir la nanoparticule lipidique.

6. Procédé de préparation d'une nanoparticule lipidique à partir d'une composition membranaire de nanoparticule lipidique selon la revendication 5, **caractérisé en ce que** le tampon PBS ne contient pas de DNase et RNase, le tampon PBS à l'étape (1) a une spécification de 1X pH=7, et le tampon PBS de l'étape (3) a une spécification de 1X pH = 7,4.

7. Procédé de préparation d'une nanoparticule lipidique à partir d'une composition membranaire de nanoparticule lipidique selon la revendication 5, **caractérisé en ce que** la solution de haute salinité est une solution de NaCl, et la concentration de NaCl dans la solution mixte à l'étape (4) est de 0,1- 1 M.

8. Procédé de préparation d'une nanoparticule lipidique à partir d'une composition membranaire de nanoparticule lipidique selon la revendication 7, **caractérisé en ce que** la concentration de NaCl dans la solution mixte est de 0,3-0,4 M.

9. Procédé de préparation d'une nanoparticule lipidique à partir d'une composition membranaire de nanoparticule lipidique selon la revendication 5, **caractérisé en ce que** le contenu est un acide nucléique.

10. Procédé de préparation d'une nanoparticule lipidique à partir d'une composition membranaire de nanoparticule lipidique selon la revendication 9, **caractérisé en ce que** l'acide nucléique est un oligonucléotide.

11. Procédé de préparation d'une nanoparticule lipidique à partir d'une composition membranaire de nanoparticule lipidique selon la revendication 5, **caractérisé en ce que** le dispositif d'ultrafiltration ou de dialyse a un seuil de coupure de poids moléculaire de 10 000 à 100 000 daltons.

12. Composition membranaire de nanoparticules lipidiques selon la revendication 1, **caractérisée en ce que** la composition membranaire comprend du DOTAP, du Egg PC, du cholestérol, du Tween 80 et du TPGS dans un rapport molaire de 25:45:20:5:5.

13. Composition membranaire de nanoparticules lipidiques selon la revendication 1, **caractérisée en ce que** la composition membranaire comprend du DOTMA, du Egg PC, du cholestérol, du Tween 80 et du TPGS dans un rapport molaire de 30:45:20:5:5.

14. Composition membranaire de nanoparticules lipidiques selon la revendication 1, **caractérisée en ce que** la composition membranaire comprend du DOTAP, du DSPC, du cholestérol, du Tween 80 et du TPGS dans un rapport molaire de 30:50:20:5:5.

15. Composition membranaire de nanoparticules lipidiques selon la revendication 1, **caractérisée en ce que** la composition membranaire comprend du DOTAP, du Egg PC, du cholestérol, du Tween 80 et du mPEG2000-DPPE dans un rapport molaire de 30:45:20:5:5.
